# EUROPEAN PATENT SPECIFICATION

(11) **EP 0 397 736 B1**
(45) Date of publication and mention of the grant of the patent: **26.06.1996**
(21) Application number: 89901675.2
(22) Date of filing: 29.12.1988
(51) Int. Cl.: G01N 21/01, G01N 33/50, G01N 33/538, G01N 33/544

(54) **APPARATUS FOR DETERMINING AN ANALYTE AND METHOD THEREFOR**
ANORDNUNG ZUR BESTIMMUNG EINES ANALYTS UND VERFAHREN DAFÜR
PROCEDE ET APPAREIL DE DETERMINATION D'UN ANALYTE

(30) Priority: 21.01.1988 US 146345
(43) Date of publication of application: 22.11.1990
(73) Proprietor: BOEHRINGER MANNHEIM CORPORATION, Indianapolis Indiana 46250 (US)
(72) Inventor: DE-LA-CROIX, Fern, Indianapolis, IN 46250-0528 (US); BERGER, Johann, Indianapolis, IN 46250-0528 (US); BUCK, Harvey, Indianapolis, IN 46250-0528 (US); GUDER, Hans-Joachim, D-6800 Mannheim 31 (DE)
(74) Representative: Kolb, Bernd, Dr.
(86) International application number: US8804697
(87) International publication number: WO8906791

(56) References cited:
- WO-A-87/02774
- WO-A-89/06792
- GB-A- 2 085 159
- US-A- 3 901 657
- US-A- 4 042 335
- US-A- 4 144 306
- US-A- 4 166 093
- US-A- 4 446 232
- US-A- 4 806 312

## Description

### FIELD OF THE INVENTION

This invention relates to apparatus useful for determining one or more components in a sample, as well as methods for determining said components.

### BACKGROUND

Chemical analysis of fluids, including body fluids such as blood, serum, urine, and so forth; water, fluid foodstuffs, etc., is often desirable and frequently necessary. Safety concerns, medical diagnosis, forensics, and other fields rely on determinations, either qualitative or quantitative, of components of fluids. These determinations, or assays, must be rapid and accurate.

A major field of clinical analysis is devoted to "dry chemistry" determinations of components in liquid samples. "Dry chemistry" refers to the apparatus used in the determinations, because the apparatus is dry to the touch. Generally, these apparatus take the form of monolayer and multilayer test strips and analytical test elements. These analytical devices have excellent storage and handling properties, and are convenient to use.

The determination of an analyte in a sample typically involves reacting the analyte with a reaction partner which undergoes some type of change following reaction, leading to a detectable signal. While the change may be caused directly by the reaction with the analyte, frequently this is not the case, as the change usually results from some property produced by the interaction between analyte and reaction product which does not exist in unreacted components.

Several types of assay have been developed which utilize the above discussed principles. One important type is the immunoenzymometric assay. This test involves the reaction of the analyte being tested with a reaction partner which carries a label. The reaction partner is contained in a test strip or other apparatus in such a way that it is non-reactive unless and until its partner analyte contacts the test strip, in the form of a fluid sample. When this happens, the analyte and reaction partner bind to each other to form a complex, which must then be determined. This is accomplished by reacting the label carried by the reaction partner with another substance so as to form a detectable signal. When the label is an enzyme, as it frequently is, the substance used is a substrate for the enzyme which, upon reaction with the enzyme, either forms a visible color or changes color. Measuring the change or amount of color gives a measure of complex, and hence of analyte.

A problem with this system, which may already be clear, is that one must, of course, have sufficient labeled reaction partner to bind essentially all of analyte in the sample. The amount of analyte, however, is not known. This thus makes it necessary to provide excess amounts of labeled reaction partner. Some of this will not react with analyte, but as it carries the label, it nonetheless forms a detectable signal. Thus, unless one separates reacted label carrier from the unreacted portion, no test result can be achieved.

Separation does, however, take place. An immunoenzymometric assay has an additional feature in that after the sample has contacted the labeled reaction partner and some of the latter has been bound, the mixture of complex and unreacted labeled reaction partner is contacted to a sample of analyte or an analyte analogue which also binds to the labeled moiety, but which is in solid phase form. This solid phase bound reactant binds to uncomplexed reaction partner, and removes it from the solution mixture. One then has a clean division of labeled partner bound to analyte, and excess labeled reaction partner bound to solid phase. Addition of substrate to either of these gives a color. If the amount of the labeled moiety with which one began is known, one measures the amount of signal either in the liquid phase, which gives a direct relationship to the amount of analyte, or in the solid phase, which provides an indirect relationship.

This type of assay involves two phases, and is thus called a heterogeneous assay. When performed using a test strip, one has the advantage of having labeled moiety, immobilized component, and reactive substance for the label all in one device. Fluid samples will diffuse through test strips, and thus the reactions will occur quickly and without the need for intervention by the investigator.

The immunoenzymometric assay is not the only type of assay used for these analytical systems. A competitive assay uses, rather than labeled binding partner, a sample of labeled analyte corresponding to the analyte being determined. The solid phase bound reactant, in these cases, is the reaction partner for the analyte and labeled analyte. If any of the analyte is in the sample, competition for the binding sites ensues. One then measures the amount of label either in the solid phase or the liquid phase in the same manner described for immunoenzymometric assays, to determine the analyte.

Yet another system, which bears some relationship to the competitive assay, is the displacement assay. In this system, labeled analyte is already bound to a solid phase. When the sample contacts the test strip containing the bound labeled analyte, some of the label will be displaced by the binding between sample analyte and solid phase bound reaction partner, and again, measurement in one of the phases is carried out.

Still another system which may be mentioned is a sandwich assay. Sandwich assays encompass a broad range of types of assays. For the invention described herein, however, a sandwich assay refers to formation of a complex between the analyte being determined, a labeled, epitopically active fragment of a monoclonal antibody, referred to as a Fab , and a nonlabeled whole monoclonal antibody, the mAb. The sandwich which forms when analyte contacts a test strip containing diffusible mAb and Fab* is mAb-An-Fab*. This sandwich contacts a solid phase containing another antibody, which binds to mAb, but not to Fab*. The result of this is to separate complex from uncomplexed Fab*, so as to permit determination in the same manner discussed supra for other systems. The prior art, which is discussed infra gives many examples of different forms of these systems.

### PRIOR ART

The following discussion presents a review of all of the art found which relates in any way to the invention disclosed herein.

Baier, et al., U.S. Patent No. 4,670,383 teaches an immunoenzymometric assay system. The assay involves adding an antibody (or fragment) which carries a label to a sample, and then addition of solid phase bound antigen to pick up excess labeled antibody. This first solid phase is removed. The liquid phase is left, and to this is added an antibody which binds either the first antibody, or to the antibody-antigen complex. It is this second step which allows for measurement of the amount of antigen in the sample.

Deutsch, et al., U.S. Patent No. 4,477,576 also involves a system similar to the Baier system. The patent discloses a method whereby labeled antibody is added to an antigen containing system. Following this the sample is combined with solid phase bound antigen. Some labeled antibody binds in solution, and some to solid phase antigen. The solid is then removed, and is immersed in a solution containing enzyme substrate. This results in a product forming which can be measured, and hence the amount of antigen may be determined.

No means are taught whereby the "mobile detectable moiety" (i.e., the unbound Ab*-Ag) is separated from substrate. In fact, the solution complex never touches the substrate.

Berke, et al., U.S. Patent No. 4 ,459,358 teaches an assay where a sample being analyzed contacts a carrier containing a binding partner which, if it does not complex, diffuses out of position. It is not solid phase bound, and is not immobilized. This diffusable product moves to a second zone, where it is picked up by an immobilized species which prevents "backwash". This patent lacks any teaching or suggestion of an immobilized reaction partner in the first zone. Nothing is taught as to how one could measure an enzyme labeled, diffused Ag (or Ag).

Liotta, U,S. Patent No. 4,446,232 teaches a device which can be used in competitive displacement or immunoenzymometric assays. In the first part (or zone) of the device, one has immobilized antigens, and labeled antibodies. When a sample containing antigen is added to the device the antigen in the sample competes with the immobilized antigen for labeled antibody. Those labeled antibodies which do not bind to the solid phase bound antigen diffuse into a second zone, where a means is present to form a detectable signal with the labeled antibodies.

Deutsch, et al., U.S. Patent Nos. 4,361,537 and 4,235,601 are related as continuation and parent. They concern a test strip ('537), and the method of using it. ('601). Only the test strip itself is considered here.

Various embodiments of test strips are disclosed, including one where a solid phase bound binding partner is used. When an immobilized form is used, however, no provision is made for measuring anything in this system but the immobilized form. This is also the case in the disclosed "rate of flow" type of system, where an immobilized form is not used. These systems rely on the difference in diffusion rate between complexes and uncomplexed moieties. Only complexed material is measured.

The '537 patent is directed to products. Deutsch, et al. rely on differential rate of diffusion between complex and uncomplexed materials. Column 16, lines 7-13 of Deutsch, et al. (either one) show this in particular. Deutsch, et al., require a retarding element (which can be the test paper itself), which slows capillary transport of a "moving element". The "moving element" is one of either the reaction product (i.e., the complex), or the first reagent (i.e., the label). "Slowing" of one to separate two implies that both are moving. In other terms, Deutsch, et al. lacks a solid phase bound partner which removed substances from solution phase.

Mochida, et al., U.S. Patent No. 4,200,436 describes an assay using a Fab or Fab' fragment which carries a label and then binds to an analyte in a sample. The complexes are separated from uncomplexed Fab, e.g., using a solid phase. Mochida, et al. does disclose the possibility of measuring non-solid phase bound material. This is done by adding a substrate which reacts with the label. There is no teaching or suggestion of a separation means or of a flow regulator.

Figueras, U.S. Patent No. 4,144,306 teaches an analytical device which contains a "reagent layer" containing a "non-diffusable material" which is a "detectable moiety" and which reacts with an analyte. This can be, e.g., a labeled antibody. When the non-diffusable material reacts with the sample, it becomes diffusible and moves to a second layer where it can be detected (the "registration layer"). Measurement can be made of material in either layer.

Behringwerke European Patent Application 186 799 teaches various test strips which can be used in different types of assays, including IEMAs, competitive, and sandwich assays. Its broadest claim sets forth an apparatus which contains a "mobile phase application zone" ("MPAZ") an "adsorptive zone" ("AZ") a "labeled reactant zone" ("RZ"), and a "solid phase zone" ("SPZ"). The claims require a specific physical relationship among these four zones.

It is noted that pages 6 and 7 of the Behringwerke disclosure require that the reagent required for detection (i.e., the enzyme substrate), be added "after the separation stage" and "after the solid phase has been adequately washed". All of the discussion at pages 6-7 require a washing step, most certainly to remove uncomplexed label from the system. As the device is characterized by fluid contact between the various zones, washing the solid phase will result in the washing liquid removing everything uncomplexed. There is no possibility of ion exchange, e.g., in the Behringwerke disclosure.

### Liberti: PCT Application PCT/US86/00668.

This patent deals with a "semi-quantitative" assay for determining whether an analyte is present in a sample over a baseline amount. This is performed by providing a test strip which contains a known amount of fixed binding complement which react with, and immobilize the analyte being determined. Also added to the solid phase are labeled analytes which will also react with the binding complement. This is used in a known amount as well. Because both react with the solid phase, one can determine how much label binds when a given amount of analyte is present in the sample. If "X" is the amount of solid phase, and "Y" is the baseline amount of analyte, then X-Y=Z, and Z is the amount of label which should bind. If the actual value obtained is less than "Z", then the analyte is present in an amount over the baseline figure. "Spillover" of labeled material can be measured. Liberti needs a device where the solid phase is present in excess as compared to the expected amount of analyte or labeled analyte. Further, Liberti clearly states that the invention is directed to a homogeneous assay, i.e.:

"The array (sic) of the present invention is described as a homogeneous array, in that no separation of bound and free specific binding pair substance is required." (page 9, lines 6-9).

Krauth, European Patent Application 122 695 (now U.S. Patent No. 4,666,866) teaches an assay which depends on the use of whole antibodies, which have two binding sites for antigens. A solid phase is used which binds only to antibodies which are not completely saturated with antigen. In other words, the solid phase will bind unbound regions of antibodies.

Greenguist, European Patent Application 212 599 (Miles)

This application (and 212 603) diverge in a direction different from that of the invention described herein. '599 teaches separating labeled, unreacted reagent which passes into the second zone of a test strip. The label, however, forms a detectable composition when immobilized and not before. Note the last three lines of claim 1 of the Greenquist application.

Greenguist, European Patent No, 212 603 (Miles)

This patent application is also directed to immobilizing non-reacted labeled material. The '603 application, however, immobilizes the material before a detectable complex forms, after which a substrate is added.

The existence of so much patent literature indicates that heterogeneous assay test strips did not solve every problem, and actually new ones were created, in many respects.

One problem which occurs in heterogeneous assay test devices of the type discussed supra is the difficulty of reading the test strip following the assay. Many of the substrates used in enzyme label systems are themselves colored. Cleavage of the substrate by the enzyme frequently causes a change amounting only to sharing of one electron over the whole substrate molecule, and results in a color shift. This can be a very small shift, i.e., from 0 - 150 nm in wavelength, with an "observable" difference of, e.g., yellow to dark yellow. The change is so small that, taken with the presence of interference from the background of unreacted substrate, the difficulty in reading the change is increased. Another problem arises when the color of the reaction product being determined is a light one. Test strips are frequently, if not always, comprised almost entirely of white paper. Light colors are lost against the white background, particularly when the amount of reacted substrate is small.

These problems have now been addressed by the invention described herein. The invention provides an apparatus which can be used in a heterogeneous assay.

Hence it is an object of the invention to provide an apparatus for determining one or more analytes in a fluid sample, which also allows for separation of one of a product of a label and its reaction component and unreacted reaction component to provide for improved analyte analyses.

It is a further object of the invention to provide an apparatus for determining one or more analytes in a fluid sample, which includes a flow regulating means for regulation of the passage of the sample through the apparatus.

It is yet a further object of the invention to provide an apparatus for determining one or more analytes in a fluid sample which provides for both separation as discussed supra, and flow regulation.

Still another object of the invention is to provide various methods for determining one or more analytes in solution, using the apparatus described herein.

How these and other objects of the invention are accomplished will be seen in the disclosure which follows.

The objects of the invention are accomplished by an apparatus for determining at least one analyte in a fluid sample, comprising several zones,
(a) a first zone containing
   (I) an immobilized reagent capable of binding to at least one of said analyte, a labelled analyte, a labelled analyte analogue, or a labelled reaction partner for said analyte, and
   (II) a removable labelled analyte, labelled analyte analogue or labelled reaction partner for said analyte, and
(b) a second zone containing a reaction component capable of reacting with the said labelled analyte, labelled analyte analogue, or labelled reaction partner

characterized in that the reaction component is mobile and forms a mobile detectable moiety when reacting with said labelled analyte, labelled analyte analogue, or labelled reaction partner and the apparatus further comprising a means for separating said mobile detectable moiety from unreacted mobile reaction component.

Furthermore the objects of the invention are accomplished by a method for determining an analyte in a fluid sample, comprising:
a) contacting said sample with non-immobilized labelled analyte, labelled analyte analogue, or labelled reaction partner for said analyte to form a first mixture;
b) contacting said first mixture to an immobilized reagent which is capable of binding to said analyte, labelled analyte, labelled analyte analogue or uncomplexed labelled reaction partner for said analyte to form a second mixture;
c) contacting said second mixture to a reactive component which is capable of forming a detectable moiety with one of said bound or unbound labelled analyte, bound or unbound labelled analyte analogue, or labelled reaction partner / analyte complex and
d) measuring the reactive component or detectable moiety as a measure of analyte in said sample, characterized in that both the reactive component and formed detectable moiety are mobile and a separated prior to the measuring step.

Furthermore the objects of the invention are accomplished by the use of an apparatus as outlined above for determining an analyte in a sample.

### Brief description of the figures

Figure 1 shows an embodiment of the invention using a separating means in the second zone.

Figure 2 shows an embodiment of the invention using both a separating means and a regulating means.

Figure 3 depicts an embodiment of the invention using a regulating means, but no separating means.

### Detailed description of preferred embodiments

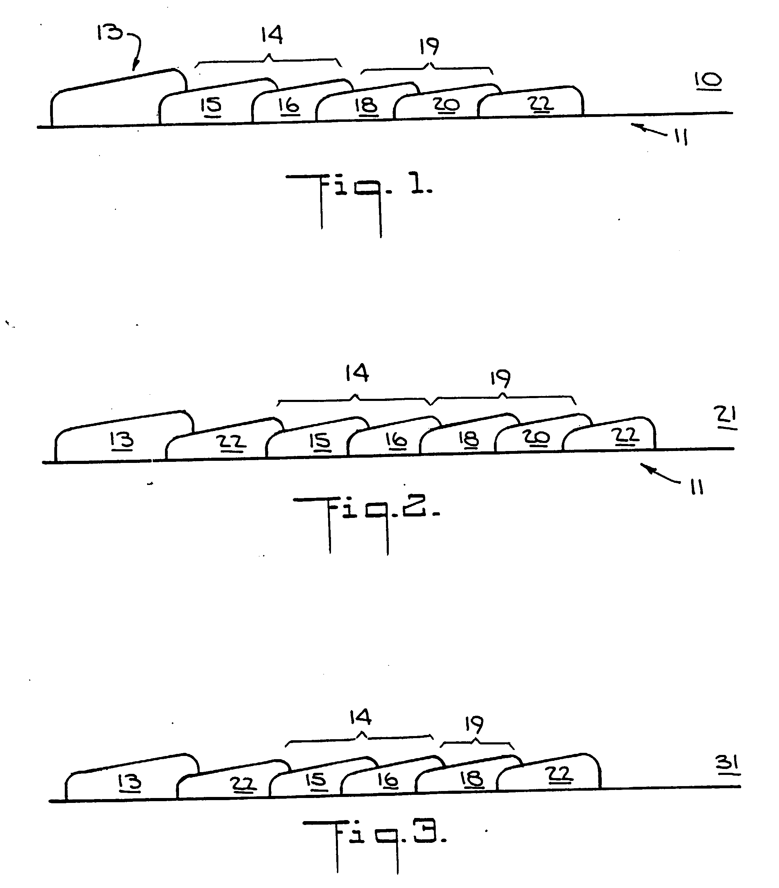

Referring now to the figures, Figure 1, shows an embodiment of the invention which uses a means for separating mobile detectable moiety from unreacted reaction component. Specifically, with reference to the figure the apparatus 10 includes a carrier foil 11, which is a backing for the additional components. This is generally, although not necessarily, made of plastic. Other than providing physical support to the strip, it has no other function.

An optional sponge 13 is provided. This feature of the device can be impregnated, with a buffer, and may receive the sample being analyzed. It will be understood, however, that the sample may be introduced at other positions in the device.

The first zone is positioned so that it receives sample which diffuses from sponge 13 when the sponge is used. If it is not,, sample may be added directly to the first zone. This zone 14 contains portions 15 and 16, corresponding to a conjugate pad 15, and a matrix 16. While this figure shows these as separate parts of the first zone, it will be understood by the skilled artisan that they may be of one piece, especially when a displacement assay is being run.

Referring to Figure 1, however, conjugate pad 15 contains the removable labelled analyte, labelled analyte analogue, or labelled reaction partner. When the sample contacts this region, either by direct contact or by diffusion, the conjugate and the sample mix, and any reactions between analyte and reaction partner take place. The mixture passes to matrix 16, which contains an immobilized form of a reactant. Generally the immobilized reagent is identical, or epitopically equivalent to the analyte being determined. When this is the case, the immobilized component must be present in an amount sufficient to bind essentially all of the labelled conjugate present in 15. This is necessary to provide for the situation where the sample contains none of the analyte being analyzed. In the case of a competitive assay, the immobilized reagents binds to both analyte and labelled analyte Again, there must be sufficient solid phase bound reagent such that if there is no analyte present, essentially all of the labeled analyte is reacted.

In a displacement assay, it will be understood that 15 and 16 will, of necessity, be of one piece because the immobilized reagent will already have bound to it the labeled analyte or analyte analogue.

Following 16 is the second zone 19. In the embodiment shown in Figure 1, this second zone contains two parts, but this is not necessarily so. Referring to the Figure substrate pad 18 contains a substrate which reacts with the label on the labeled component to form a detectable signal. This may be, but need not be, an enzyme substrate. Trapper pad 20, which is key to the invention, is in fluid contact with the substrate pad 18, or, if 18 and 20 constitute one piece, this one piece second zone is in fluid contact with the first zone. The trapper pad contains a means, such as ionic exchange paper, which traps either the reaction product of the label and substrate, or unreacted substrate.

Finally, in fluid contact with the second zone is the waste pad 22, which is adapted for receiving excess fluid. Further, it absorbs any materials which may be removed when the test strip is washed. The waste pad 22 can, alternatively, be used as a measuring point. When separation of detectable moiety and unreacted reaction component takes place in the second zone, the element which is not trapped can be washed into the waste pad. This element, rather than the trapped element, can be measured as well as, or in preference to, the trapped element.

Another embodiment is shown in Figure ②. This embodiment is identical to that shown in Figure 1, except that it adds regulating means 22 to the device 21. The regulating means may contain substances, such as a buffer, which help to regulate flow of the analyte containing sample. Component 22 may be placed at various points in the apparatus. For example, it may be positioned in the first zone itself, such as between 15 and 16 of Figure 2; it may be between 16 and 18 in this figure, in the second zone, for example, by being positioned between Figures 18 and 20, or even at the end of the second zone and before the waste pad. The regulating means can be made of various substances, such as paper which possesses capillarity properties which differs from at least one of its neighbors.

Figure ③ shows yet another of the various embodiments encompassed by this invention. The embodied device 31 is identical to that of Figure 2, but it lacks the trapper zone 20. The device depicted here regulates flow of sample therethrough, with concentration of detectable moiety or unreacted reaction component.

The apparatus shown in Figures 1-3 are all usable for determining one or more analytes in any sample to be analyzed. For example, if the analysis being undertaken is to determine the presence of illicit drugs, a urine sample is taken from the subject and either sponge 13 is dipped therein, or an amount of urine is added directly to the conjugate pad 15. The dose is small. When urine, e,g., is added directly to the first zone, 50 1 is sufficient. When the sponge is used, this generally holds about 1 ml.

Optionally additional material such as a "run buffer" or a washing solution may be used. In such cases, this material may be added at the same point as the sample or at some different point or points in the apparatus.

When the sponge is used, alone or with a buffer containing regulating means, the sample diffuses through 13 and 17, as provided in the device, until it reaches first zone component 15. Of course, if sample is added here directly, the diffusion through 13 and 17 does not occur. In the first zone, the sample encounters enzyme labeled antibodies against the drug or drugs to be identified. If more than one drug is being assayed, each type of antibody may carry a different label but need not. Reactions occur between whatever drugs are present in the sample and the labeled antibodies. These complexes, plus any uncomplexed labeled antibody flows to that portion of the first zone 16 which contains immobilized reagents, in this case, immobilized drug or drugs which correspond to those being assayed. The immobilized drugs react with the uncomplexed antibodies, removing these from solution. The complex of drugs and labeled antibodies flow into the second zone, where substrate reacts with any label carried therein. If a separating means is employed, as in Figures 1 and 2, this acts either to separate the reaction product of label and substrate from unreacted substrate, or unreacted substrate from the reaction product. When more than one drug is being tested, e.g., additional systems of substrate and enzyme are provided, each of which can be reacted and separated differentially. One then determines either reaction product or unreacted substrate in either the waste pad or the second zone. The determination step can be preceded by a washing step, if appropriate.

While the foregoing example was given for illicit drugs, different systems will be seen to be readily available to the skilled artisan. Among these substances which can be assayed include antibodies, antigens, and naturally occurring substances such as hormones, biological byproducts, and so forth. Viral infections, such as HIV, microbial or parasitic infections, such as Chlamydia, may be assayed as well. The test strips can be used to determine substances like glucose, various enzymes such as alpha amylase, and so forth.

Further particular embodiments will be seen from the following:

### Example 1

A test strip is prepared for analysis of thyroxin (T4) in a fluid. The test strip is configured as is the device of Figure 2. The first zone contains antibodies to T4 labeled with the enzyme beta-galactosidase (4U/ml), and T4-succinimide ester which is immobilized onto CnBr activated paper (3512, Schleicher and Schuell). The regulating means contains phosphate buffered saline (PBS) on Whatman 54 paper, and chlorophenol-red-beta-D-galactopyranoside (CPRG), a substrate for beta galactosidase, is impregnated into the second zone, together with an ion exchange trap (DE81, Whatman paper). CPRG, when unreacted, is yellow. Upon reaction with beta galactosidase, the reaction product is purple; however, when a separating means such as the ion exchange paper is not used, the resulting mixture of reaction product and unreacted CPRG is a muddy brown color. Neither the purple color indicative of the reaction nor the yellow color showing unreacted substrate is easily discernable. When the ion exchange paper is used, however, the purple color of the reaction product is concentrated and discernable from the yellow CPRG. If desired, unreacted yellow can be removed in a washing step.

### Example 2

Using the embodiment shown in Figure 1, a test device is prepared to determine phenobarbital in a biological fluid.

Into the first zone are incorporated antibodies to phenobarbital conjugated to horseradish peroxidase, and immobilized phenobarbital-succinimide ester, on CnBr activated paper as in Example 1. The second zone contains the horseradish peroxidase substrate vanillin azine (0.5 mg/ml) which, in unreacted form, is yellow. The trap is designed to pick up unreacted vanillin azine, but to pass the reaction product of horseradish peroxidase and vanillin azine, which is purple, into the waste zone.

### Example 3

This example uses the device shown in Figure ③ and is designed for the determination of human chorionic gonadotropin (hCG), a pregnancy hormone. A conjugate of hCG specific antibody and beta galactosidase is incorporated into the first zone, together with hCG immobilized onto CnBr activated paper. The flow regulating means contains PBS, and the second zone contains resorufin-beta-D-galactopyranoside. Those complexes of hCG and conjugate which flow to the second zone react with the resorufin substrate forming an observable fuschia color.

It will be understood by those skilled in the art that the particular materials used for the flow regulating means or the separating means can and will vary. Different factors, such as the substrate used, the analyte being assayed, and the label determine what materials are used. Similarly, the substrate must be chosen so as to give some observable or detectable result when reacted with the label. Typically, the reaction is between an enzyme label and a substrate, but this is not the only possibility. For example, the label can be a fluorescent substance, and the reactive component a substance which caps or quenches the fluorescence. The label may itself be a cleavable substance and the reactive component a substance which performs the cleavage, leading to color formation.

The apparatus described supra, as will be seen, comprises a plurality of zones, the first of which contains the reactive components for the analyte to be determined. Depending on the test system being used, some facets of the zone may vary. Typically, this zone will contain at least one immobilized reagent which binds to the analyte or analyte being determined. Typically, this immobilized reagent is a member of an antigen-antibody complex. This need not always be the case, however, as this immobilized reagent may be, e.g., protein A, a streptavidin-bictin complex, or any of the materials familiar to the skilled artisan. This zone also contains at least one of a labeled analyte, labeled analyte analogue, or labeled reaction partner for the analyte being determined. The choice of what labeled moiety is used depends upon various parameters, such as reagent availability and cost. The label, typically, is an enzyme, but this is not essential. Systems are also envisioned where the label is, e.g., a fluorescent or radioactive substance, a portion of an enzyme which later binds to a complement to form a whole, operational enzyme, and other materials which will be recognized by the skilled artisan. When multiple analytes are being determined, of course, the different labeled analytes and so forth will differ. The label itself, however, can be the same or different for the plurality of labeled moieties. This is so because, even if the reaction between label and reactive component is the same with formation of the same detectable moiety, the difference in the analyte means that the different substances may separate differently. When a flow regulating means is incorporated here, this means may be any substance which has flow properties which differ from any part of the device. The flow regulating means may also have incorporated therein any of the various reagents described supra.

The second zone contains the reaction component which reacts with the label to form the detectable moiety. As was explained, supra, typically this reaction component is an enzyme substrate, but it need not be, and attention is drawn to the foregoing discussion for other materials which may be used. In the case where multiple analytes are being determined, different reaction components may be used when the labels differ, but, as was pointed out, supra, they do not need to be.

The separation means is preferably an ionic exchange means, such as ionic exchange paper, which has particular attraction for one of the detectable moiety or unreacted reaction component as compared to the other. Different types of separation means can be used as well, such as materials which, in connection with the detectable moiety and unreacted reaction component, have different degrees of hydrophobic interaction with the two. It is important to recognize that the interaction between separating means and the substance which separates out need not be a "chemical reaction" in the classic sense, i.e., that the separated substance undergoes chemical reaction which somehow alters the substance.

Various embodiments of the devices and methods described and claimed herein will of course be evident to the skilled artisan. The examples given herein are in no way to be construed as limitative of the broad disclosure.

While there have been described what are at present considered to be the preferred embodiments of this invention, it will be obvious to one skilled in the art that various changes and modifications may be made therein without departing from the invention, and it is, therefore, aimed to cover all such changes and modifications as fall within the scope of the invention.

## Claims

1. Apparatus (10, 21, 31) for determining at least one analyte in a fluid sample, comprising several zones,
(a) a first zone (14) containing
(I) an immobilized reagent capable of binding to at least one of said analyte, a labeled analyte, a labeled analyte analogue, or a labeled reaction partner for said analyte, and
(II) a removable labeled analyte, labeled analyte analogue or labeled reaction partner for said analyte, and
(b) a second zone (19) containing a reaction component capable of reacting with the said labeled analyte, labeled analyte analogue, or labeled reaction partner
characterized in that the reaction component is mobile and forms a mobile detectable moiety when reacting with said labeled analyte, labeled analyte analogue, or labeled reaction partner and the apparatus further comprising a means for separating said mobile detectable moiety from unreacted mobile reaction component.

2. Apparatus of claim 1 further comprising at least one means (22) incorporated therein which regulates movement of fluid sample introduced thereto, wherein said means possesses flow rate properties which differ from those of at least a portion of said apparatus.

3. Apparatus of claim 1 or 2, wherein said first and second zones (14,19) are in at least partial fluid contact with each other.

4. Apparatus of claim 1 or 2, wherein said first zone (14) comprises two parts, a first part (15) which contains one of a labeled reaction partner for said analyte, a labeled analyte or a labeled analyte analogue and a second part (16) which contains an immobilized reagent which binds to labeled reaction partner unbound to said analyte, labeled analyte analogue, or labeled analyte.

5. Apparatus of claim 1 or 2, wherein said second zone (19) comprises two parts, a first part (18) of which contains said component and a second part (20) which contains said separating means.

6. Apparatus of claim 1 or 2, wherein said first zone (14) comprises two parts, a first part (15) which contains a labeled reaction partner for said analyte and a second part (16) containing an immobilized reagent which binds to labeled reaction partner unbound to said analyte, and said second zone (19) comprises two parts, a first part (18) of which contains said compound for reacting with said unbound reaction partner and a second part (20) which contains said separating means.

7. Apparatus of claim 2, wherein said flow regulating means (22) is incorporated in said first zone (14).

8. Apparatus of claim 2, wherein said flow regulating means (22) is incorporated in said second zone (19).

9. Apparatus of claim 2, wherein said regulating means (22) is positioned between said first and second zones (14, 19).

10. Apparatus of claim 2, wherein said flow regulating means (22) is positioned before said first zone (14).

11. Apparatus of claim 1 or 2, wherein said separating means (20) is an ion exchange paper with affinity for said detectable moiety or reaction component.

12. Apparatus of claim 1 or 2, wherein said first zone (14) further comprises at least one labeled receptor, wherein said receptor specifically binds to an analyte in a fluid sample to be analyzed.

13. Apparatus of claim 1 or 2, wherein said first zone (14) further comprises at least one labeled analyte or labeled analyte analogue wherein said labeled analyte is identical to an analyte sample to be determined or said labeled analyte analogue is a derivative of said analyte.

14. Apparatus of claim 1 or 2, wherein said first zone (14) further comprises a plurality of labeled receptors each of said receptors specifically binding to a particular analyte to be determined.

15. Apparatus of claim 1 or 2, wherein said first zone (14) further comprises a plurality of labeled analytes or labeled analyte analogues, each of said labeled analytes or labeled analyte analogues corresponding to an analyte to be determined.

16. Apparatus of claim 12, wherein said labeled receptor is an antibody or fragment thereof

17. Apparatus of claim 14, wherein said labeled receptors are antibodies or fragments thereof.

18. Apparatus of claim 14, wherein said second zone (19) further comprises a plurality of reaction components, each of said reaction components reacting with one of said plurality of labeled receptors.

19. Apparatus of claim 15, wherein said second zone (19) further comprises a plurality of reaction components, each of said reaction components reacting with one of said plurality of labeled analytes or labeled analyte analogues.

20. Apparatus of claim 2, wherein said second zone (19) comprises two parts, a first part (18) of which contains said component and a second part (22) which contains said flow regulation means.

21. Apparatus of claim 2 wherein said flow regulating means (22) is filter paper.

22. Apparatus of claim 2, wherein said first zone (14) comprises two parts, a first part (15) which contains a labeled reaction partner for said analyte and a second part (16) containing an immobilized reagent which binds to labeled reaction partner unbound to said analyte, and said second (19) zone comprises two parts, a first part (18) of which contains said compound for reacting with said unbound labeled reaction partner and a second part (20) which contains said separating means.

23. Method for determining an analyte in a fluid sample, comprising:
a) contacting said sample with non-immobilized labelled analyte, labelled analyte analogue, or labelled reaction partner for said analyte to form a first mixture;
b) contacting said first mixture to an immobilized reagent which is capable of binding to said analyte, labelled analyte, labelled analyte analogue or uncomplexed labelled reaction partner for said analyte to form a second mixture;
c) contacting said second mixture to a reactive component which is capable of forming a detectable moiety with one of said bound or unbound labelled analyte, bound or unbound labelled analyte analogue, or labelled reaction partner / analyte complex and
d) measuring the reactive component or detectable moiety as a measure of analyte in said sample,
characterized in that both the reactive component and formed detectable moiety are mobile and are separated prior to the measuring step.

24. Use of the apparatus of claim lor 2 for determining an analyte in a sample.

## Patentansprüche

1. Vorrichtung (10, 21, 31) zur Bestimmung wenigstens eines Analyten in einer flüssigen Probe, umfassend mehrere Zonen,
a) eine erste Zone (14), enthaltend
(I) ein immobilisiertes Reagens, das imstande ist, an wenigstens ein Mitglied aus der Gruppe Analyt, markierter Analyt, markiertes Analytanalogon oder markierter Reaktionspartner für den Analyt zu binden, und
(II) einen abtrennbaren markierten Analyt, ein markiertes Analytanalogon oder einen markierten Reaktionspartner für den Analyt, und
b) eine zweite Zone (19), enthaltend eine Reaktionskomponente, die imstande ist, mit dem markierten Analyt, dem markierten Analytanalogon oder dem markierten Reaktionspartner zu reagieren,
dadurch gekennzeichnet, daß die Reaktionskomponente beweglich ist und eine bewegliche nachweisbare Einheit bildet, wenn sie mit dem markierten Analyt, dem markierten Analytanalogon oder dem markierten Reaktionspartner reagiert, wobei die Vorrichtung des weiteren ein Hilfsmittel zur Abtrennung der beweglichen nachweisbaren Einheit von der unumgesetzten beweglichen Reaktionskomponente umfaßt.

2. Vorrichtung nach Anspruch 1, des weiteren umfassend wenigstens ein darin eingebrachtes Hilfsmittel (22), das die Bewegung einer dazu eingeführten flüssigen Probe reguliert, wobei das Hilfsmittel Fließgeschwindigkeitseigenschaften besitzt, die sich von denjenigen wenigstens eines Teils der Vorrichtung unterscheiden.

3. Vorrichtung nach Anspruch 1 oder 2, wobei die erste und zweite Zone (14, 19) in wenigstens teilweisem Flüssigkeitskontakt miteinander stehen.

4. Vorrichtung nach Anspruch 1 oder 2, wobei die erste Zone (14) zwei Teile umfaßt, einen ersten Teil (15), der einen markierten Reaktionspartner für den Analyt, einen markierten Analyt oder ein markiertes Analytanalogon enthält, und einen zweiten Teil (16), der ein immobilisiertes Reagens enthält, das an den markierten, nicht an den Analyten, das markierte Analytanalogon oder den markierten Analyten gebundenen Reaktionspartner bindet.

5. Vorrichtung nach Anspruch 1 oder 2, wobei die zweite Zone (19) zwei Teile umfaßt, einen ersten Teil (18), der die Komponente enthält, und einen zweiten Teil (20), der das Trennhilfsmittel enthält.

6. Vorrichtung nach Anspruch 1 oder 2, wobei die erste Zone (14) zwei Teile umfaßt, einen ersten Teil (15), der einen markierten Reaktionspartner für den Analyt enthält, und einen zweiten Teil (16), der ein immobilisiertes Reagens enthält, das an den markierten, nicht an den Analyten gebundenen Reaktionspartner bindet, und die zweite Zone (19) zwei Teile umfaßt, einen ersten Teil (18), der die Verbindung für die Umsetzung mit dem nichtgebundenen Reaktionspartner enthält, und einen zweiten Teil (20), der das Trennhilfsmittel enthält.

7. Vorrichtung nach Anspruch 2, wobei das Hilfsmittel zur Durchflußregulierung (22) in die erste Zone (14) eingebracht ist.

8. Vorrichtung nach Anspruch 2, wobei das Hilfsmittel zur Durchflußregulierung (22) in die zweite Zone (19) eingebracht ist.

9. Vorrichtung nach Anspruch 2, wobei sich das Hilfsmittel zur Durchflußregulierung (22) zwischen der ersten und zweiten Zone (14, 19) befindet.

10. Vorrichtung nach Anspruch 2, wobei sich das Hilfsmittel zur Durchflußregulierung (22) vor der ersten Zone (14) befindet.

11. Vorrichtung nach Anspruch 1 oder 2, wobei das Trennhilfsmittel (20) ein Ionenaustauschpapier mit Affinität für die nachweisbare Einheit oder die Reaktionskomponente ist.

12. Vorrichtung nach Anspruch 1 oder 2, wobei die erste Zone (14) des weiteren wenigstens einen markierten Rezeptor umfaßt, wobei der Rezeptor spezifisch an einen Analyten in einer zu analysierenden flüssigen Probe bindet.

13. Vorrichtung nach Anspruch 1 oder 2, wobei die erste Zone (14) des weiteren wenigstens einen markierten Analyten oder ein markiertes Analytanalogon umfaßt, wobei der markierte Analyt mit einer zu bestimmenden Analytprobe identisch ist, oder das markierte Analytanalogon ein Derivat des Analyten ist.

14. Vorrichtung nach Anspruch 1 oder 2, wobei die erste Zone (14) des weiteren mehrere markierte Rezeptoren umfaßt, wobei jeder der Rezeptoren spezifisch an einen speziellen, zu bestimmenden Analyten bindet.

15. Vorrichtung nach Anspruch 1 oder 2, wobei die erste Zone (14) des weiteren mehrere markierte Analyten oder markierte Analytanaloga umfaßt, wobei die markierten Analyten oder markierten Analytanaloga jeweils einem zu bestimmenden Analyten entsprechen.

16. Vorrichtung nach Anspruch 12, wobei der markierte Rezeptor ein Antikörper oder ein Fragment desselben ist.

17. Vorrichtung nach Anspruch 14, wobei die markierten Rezeptoren Antikörper oder Fragmente derselben sind.

18. Vorrichtung nach Anspruch 14, wobei die zweite Zone (19) des weiteren mehrere Reaktionskomponenten umfaßt, wobei jede der Reaktionskomponenten mit einem der mehreren Rezeptoren reagiert.

19. Vorrichtung nach Anspruch 15, wobei die zweite Zone (19) des weiteren mehrere Reaktionskomponenten umfaßt, wobei jede der Reaktionskomponenten mit einem der mehreren markierten Analyten oder markierten Analytanaloga reagiert.

20. Vorrichtung nach Anspruch 2, wobei die zweite Zone (19) zwei Teile umfaßt, einen ersten Teil (18), der die Komponente enthält, und einen zweiten Teil (22), der das Hilfsmittel zur Durchflußregulierung enthält.

21. Vorrichtung nach Anspruch 2, wobei das Hilfsmittel zur Durchflußregulierung (22) Filterpapier ist.

22. Vorrichtung nach Anspruch 2, wobei die erste Zone (14) zwei Teile umfaßt, einen ersten Teil (15), der einen markierten Reaktionspartner für den Analyt enthält, und einen zweiten Teil (16), der ein immobilisiertes Reagens enthält, das an den markierten, nicht an den Analyten gebundenen Reaktionspartner bindet, und die zweite Zone (19) zwei Teile umfaßt, einen ersten Teil (18), der die Verbindung für die Umsetzung mit dem nichtgebundenen markierten Reaktionspartner enthält, und einen zweiten Teil (20), der das Trennhilfsmittel enthält.

23. Methode zur Bestimmung eines Analyten in einer flüssigen Probe, umfassend:
a) Zusammenbringen der Probe mit einem nichtimmobilisierten markierten Analyt, markierten Analytanalogon oder markierten Reaktionspartner für den Analyt, um eine erste Mischung zu bilden;
b) Zusammenbringen der ersten Mischung mit einem immobilisierten Reagens, das imstande ist, an den Analyt, den markierten Analyt, das markierte Analytanalogon oder den nichtkomplexierten markierten Reaktionspartner für den Analyt zu binden, um eine zweite Mischung zu bilden;
c) Zusammenbringen der zweiten Mischung mit einer reaktionsfähigen Komponente, die imstande ist, mit gebundenem oder ungebundenem markiertem Analyt, gebundenem oder ungebundenem markierten Analytanalogon oder markiertem Reaktionspartner/Analytkomplex eine nachweisbare Einheit zu bilden, und
d) Messen der reaktionsfähigen Komponente oder der nachweisbaren Einheit als ein Maß für den Analyten in der Probe,
dadurch gekennzeichnet, daß sowohl die reaktionsfähige Komponente als auch die gebildete nachweisbare Einheit beweglich sind und vor dem Meßschritt getrennt werden.

24. Verwendung der Vorrichtung nach Anspruch 1 oder 2 zur Bestimmung eines Analyten in einer Probe.

## Revendications

1. Dispositif (10, 21, 31) pour la détermination d'au moins un analyte dans un échantillon fluide, comprenant plusieurs zones,
(a) une première zone (14) contenant
(I) un réactif immobilisé capable de se lier à au moins un parmi ledit analyte, un analyte marqué, un analogue d'analyte marqué, ou un partenaire de réaction marqué pour ledit analyte, et
(II) un analyte marqué pouvant être retiré, un analogue d'analyte marqué ou un partenaire de réaction marqué pour ledit analyte, et
(b) une deuxième zone (19) contenant un composant de réaction capable de réagir avec ledit analyte marqué, analogue d'un analyte marqué, ou partenaire de réaction marqué
caractérisé en ce que le composant de réaction est mobile et forme une fraction détectable mobile lorsqu'on le fait réagir avec ledit analyte marqué, analogue d'analyte marqué, ou partenaire de réaction marqué et le dispositif comprenant de plus un moyen de séparation de ladite fraction détectable mobile du composant de réaction mobile n'ayant pas réagi.

2. Dispositif selon la revendication 1, comprenant de plus au moins un moyen (22) qui y est incorporé, qui règle le déplacement d'un échantillon liquide que l'on y a introduit, dans lequel ledit moyen possède des propriétés de vitesse d' écoulement qui diffèrent de celles d'au moins une partie dudit dispositif.

3. Dispositif selon la revendication 1 ou 2, dans lequel lesdites première et deuxième zones (14,19) sont au moins en contact partiel fluide l'un avec l'autre.

4. Dispositif selon la revendication 1 ou 2, dans lequel ladite première zone (14) comprend deux parties, une première partie (15) qui contient un parmi un partenaire de réaction marqué pour ledit analyte, un analyte marqué ou un analogue d'analyte marqué et une seconde partie (16) qui contient un réactif immobilisé qui se lie au partenaire de réaction marqué non lié audit analyte, analogue d'analyte marqué, ou analyte marqué.

5. Dispositif selon la revendication 1 ou 2, dans lequel ladite deuxième zone (19) comprend deux parties, dont une première partie (18) contient ledit composant et une deuxième partie qui contient ledit moyen de séparation.

6. Dispositif selon la revendication 1 ou 2, dans lequel ladite première zone (14) comprend deux parties, une première partie (15) qui contient un partenaire de réaction marqué pour ledit analyte et une deuxième partie (16) comprenant un réactif immobilisé qui se lie au partenaire de réaction marqué non lié audit analyte, et ladite deuxième zone (19) comprend deux parties dont une première partie (18) contient ledit composé pour la réaction avec ledit partenaire de réaction non lié et une deuxième partie (20) qui contient ledit moyen de séparation.

7. Dispositif selon la revendication 2, dans lequel ledit moyen de régulation de l'écoulement (22) est incorporé dans ladite première zone (14).

8. Dispositif selon la revendication 2, dans lequel ledit moyen de régulation de l'écoulement (22) est incorporé dans ladite deuxième zone (19).

9. Dispositif selon la revendication 2, dans ledit moyen de régulation est positionné entre lesdites première et deuxième zones (14, 19).

10. Dispositif selon la revendication 2, dans lequel ledit moyen de régulation de l'écoulement (22) est positionné avant ladite première zone (14).

11. Dispositif selon la revendication 1 ou 2, dans lequel ledit moyen de séparation (20) est un papier échangeur d'ion avec une affinité pour ladite fraction détectable ou composant de réaction.

12. Dispositif selon la revendication 1 ou 2, dans lequel ladite première zone (14) comprend encore au moins un récepteur marqué, dans lequel ledit récepteur se lie spécifiquement à un analyte dans un échantillon fluide à analyser.

13. Dispositif selon la revendication 1 ou 2, dans lequel ladite première zone (14) comprend encore au moins un analyte marqué ou un analogue d'analyte marqué dans lequel ledit analyte marqué est identique à un échantillon d'analyte à déterminer ou ledit analogue d'analyte marqué est un dérivé dudit analyte.

14. Dispositif selon la revendication 1 ou 2, dans lequel ladite première zone (14) comprend encore une pluralité de récepteurs marqués, chacun desdits récepteurs se liant de manière spécifique à un analyte particulier à déterminer.

15. Dispositif selon la revendication 1 ou 2, dans lequel ladite première zone (14) comprend encore une pluralité d'analytes marqués ou d'analogues d'analyte marqué, chacun desdits analytes marqués ou analogues d'analyte marqué correspondant à un analyte à déterminer.

16. Dispositif selon la revendication 12, dans lequel ledit récepteur marqué est un anticorps ou fragment de celui-ci.

17. Dispositif selon la revendication 14, dans lequel lesdits récepteurs marqués sont des anticorps ou des fragments de ceux-ci.

18. Dispositif selon la revendication 14, dans lequel ladite deuxième zone (19) comprend encore une pluralité de composants de réaction, chacun desdits composants de réaction réagissant avec l'un de ladite pluralité de récepteurs marqués.

19. Dispositif selon la revendication 15, dans lequel ladite deuxième zone (19) comprend encore une pluralité de composants de réaction, chacun desdits composants de réaction réagissant avec un de ladite pluralité d'analytes marqués ou d'analogues d'analyte marqués.

20. Dispositif selon la revendication 2, dans lequel ladite deuxième zone (19) comprend deux parties, dont une première partie (18) contient ledit composant et une deuxième partie (22) qui contient ledit moyen de régulation de l'écoulement.

21. Dispositif selon la revendication 2, dans lequel ledit moyen de régulation de l'écoulement (22) est du papier filtre.

22. Dispositif selon la revendication 2, dans lequel ladite première zone (14) comprend deux parties, une première partie (15) qui contient un partenaire de réaction marqué pour ledit analyte et une deuxième partie (16) contenant un réactif immobilisé qui se lie au partenaire de réaction marqué non lié audit analyte et une dite deuxième zone (19) comprend deux parties, dont une première partie (18) contient ledit composé pour réagir avec ledit partenaire de réaction marqué non lié et une deuxième partie (20) qui contient ledit moyen de séparation.

23. Procédé de détermination d'un analyte dans un échantillon fluide, consistant:
a) à mettre en contact ledit échantillon avec un analyte marqué, un analogue d'analyte marqué, ou un partenaire de réaction marqué non immobilisé pour ledit analyte afin de former un premier mélange;
b) à mettre en contact ledit premier mélange avec un réactif immobilisé qui est capable de se lier audit analyte, analyte marqué, analogue d'analyte marqué ou partenaire de réaction marqué non complexé pour ledit analyte afin de former un deuxième mélange;
c) à mettre en contact ledit deuxième mélange avec un composant réactif qui est capable de former une fraction détectable avec l'un parmi ledit analyte marqué lié ou non lié, l'analogue d'analyte marqué lié ou non lié, ou le partenaire de réaction marqué/complexe analyte et
d) à mesurer le composant réactif ou la fraction détectable en tant que mesure de l'analyte dans ledit échantillon,
caractérisé en ce que à la fois le composant réactif et la fraction détectable formée sont mobiles et sont séparés avant l'étape de mesure.

24. Utilisation du dispositif selon la revendication 1 ou 2 pour la détermination d'un analyte dans un échantillon.
